Europäisches Patentamt

European Patent Office

Office européen des brevets

(11) Publication number: **0 277 216 B1**

# EUROPEAN PATENT SPECIFICATION

(45) Date of publication of patent specification: **11.08.93** (51) Int. Cl.5: **C12N 9/54**, C11D 3/386

(21) Application number: **87905616.6**

(22) Date of filing: **14.08.87**

(86) International application number:
**PCT/DK87/00101**

(87) International publication number:
**WO 88/01293 (25.02.88 88/05)**

(54) **ALKALINE PROTEASE DERIVED FROM BACILLES PRODUCTION AND USE THEREOF.**

(30) Priority: **14.08.86 DK 3865/86**

(43) Date of publication of application:
**10.08.88 Bulletin 88/32**

(45) Publication of the grant of the patent:
**11.08.93 Bulletin 93/32**

(84) Designated Contracting States:
**AT BE CH DE FR GB IT LI LU NL SE**

(56) References cited:
EP-A- 0 220 921      DE-A- 2 005 232
GB-A- 1 243 784      US-A- 3 674 643
US-A- 3 723 250      US-A- 3 748 233
US-A- 3 827 938      US-A- 3 905 869
US-A- 4 002 572      US-A- 4 052 262
US-A- 4 480 037

Kirk-Othmer "Encyclopedia of Chemical
Technology" Volume 9, 1980, John Wiley &
Sons, New York, p. 138-147

(73) Proprietor: **NOVO NORDISK A/S**
**Novo Allé**
**DK-2880 Bagsvaerd(DK)**

(72) Inventor: **OUTTRUP, Helle**
**Syvendehusvej 46**
**DK-2750 Ballerup(DK)**
Inventor: **DAMBMANN, Claus**
**Hoje Gladsaxe 61, 7.th.**
**DK-2860 Soborg(DK)**
Inventor: **BRANNER, Sven**
**Ved Smedebakken 7A**
**DK-2800 Lyngby(DK)**

(74) Representative: **Knudsen, Sten Lottrup et al**
**c/o Novo Nordisk A/S, Patent Department,**
**Novo Allé**
**DK-2880 Bagsvaerd (DK)**

Chemical Abstracts, vol, 81, 1974, abstract no.59820t, Su, Yuan-Chi et al.

Chemical Abstracts, vol. 102, 1985, abstract no.202605m, NL 8302790, 8 Aug. 1983

Chemical Abstracts, vol. 96, 1982, abstract no.64654f, Dambmann et al.

Chemical Abstracts, vol. 72, 1970, abstract no.14098m

## Description

The present invention is directed to a novel alkaline protease preparation having enhanced oxidation stability, to a method for the preparation thereof, and to its use as an additive for cleaning agents. Typical cleaning agents are detergent compositions for laundering.

## BACKGROUND OF THE INVENTION

Proteolytic enzymes produced by cultivation of members of the genus Bacillus in suitable nutrient media are widely used in detergent compositions. Examples of such commercial protease products are ALCALASE, ESPERASE and SAVINASE (these are registered trade marks), all supplied by NOVO Industri A/S (now Novo Nordisk A/S), Denmark. These exhibit adequate storage stability in the presence of the usual components of detergent compositions, such as sequestering agents, surfactants and even bleaching agents of the peroxy type, provided that they are suitably protected from their physical environment in the detergent composition, for example by their incorporation into suitably coated particles.

However, in the laundering process where the solubilized enzyme is exposed to the same agents in solution, stability may become critical in the sense that the enzyme is prone to destruction prior to the completion of its intended stain-removing action, particularly at elevated temperatures in the presence of bleaching agents of the peroxy type, such as percarbonate, perborate, and persulfate salts. Since all Bacillus-derived proteases known in the art exhibit an appreciable instability at laundering temperatures exceeding 50°C in the presence of a peroxy compound it is often recommended to prolong the washing programme by including a holding time at lower temperature when detergent protease compositions are used.

Attempts to enhance the stability of Bacillus derived serine proteases towards oxidizing agents by applying the technique of site specific mutation to the corresponding genes is known in the art, e.g. from European Patent Publication No. 0 130 756 which discloses stabilized subtilisins by substituting the methionine residue next to the active-site-serine residue with amino acid residues which are less prone to oxidation. However, the obstacles associated with the adaption of recombinant DNA strategies to large scale manufacturing processes are well-known, the most frequent ones being instability of the engineered microorganism through loss of genetic material and lower yields compared with those obtained from the wild strain or its conventional mutants.

As far as the inventors hereof are aware the prior art is silent about non-engineered Bacillus derived serine proteases exhibiting an enhanced stability towards oxidation as compared with commercial Bacillus proteases.

The object of the present invention is to overcome the above-mentioned instability problems encountered with the Bacillus-derived alkaline protease preparations known heretofore. According to the invention this object has been met in that it has surprisingly been found that a number of Bacillus strains belonging to what is presently defined as the same novel species produce alkaline protease which exhibits a remarkably enhanced stability towards bleaching agents of the peroxy type conventionally used for laundering.

Normally, the alkaline protease produced by the novel Bacillus species of this invention will contain three main constituent, mutually distinguishable alkaline proteases (herein designated A, B and C) often in proportions, measured in protease activity units, of roughly one third of each. However, variant strains may lack the capability of producing at least one of these constituent proteases. Fortuitously, all three constituent proteases show similar detergency and stability characteristics.

## SUMMARY OF THE INVENTION

Thus, according to its first aspect the present invention provides a protease preparation comprising alkaline protease derivable from a novel alkalophilic Bacillus species defined by the strain NCIB 12288 and NCIB 12289 which alkaline protease retains a residual activity measured in Casein Protease Units (CPU) after 30 minutes at pH 9.5 in a solution of 1.75 g/l of sodium tripolyphosphate (STPP), 1.0 g/l of sodium perborate and 0.1 g/l of tetraacetylethylenediamine (TAED) of at least 80% and 60% at temperatures of 40°C and 50°C, respectively, of the residual activity at 25°C in the absence of sodium perborate and TAED, the protease having a molecular weight (SPS-PAGE) of about 41.5 kD and an isoelectric point (IEF) of about 9.3, a molecular weight (SPS-PAGE) of about 28.5 kD and an isoelectric point (IEF) of about 8.8 or a molecular weight (SPS-PAGE) of about 30.0 kD and an isoelectric point (IEF) of about 5.2, the protease showing immunochemical non-identity to known alkaline Bacillus proteases, namely "ALCALASE" (derived from Bacillus licheniformis, with subtilisin Carlsberg as the active component), "SAVINASE" and "ESPERASE" (prepared from alkalophilic Bacillus sp. according to U.S. Patent No. 3723250) and "API-21" (U.S. Patent No. 4480037).

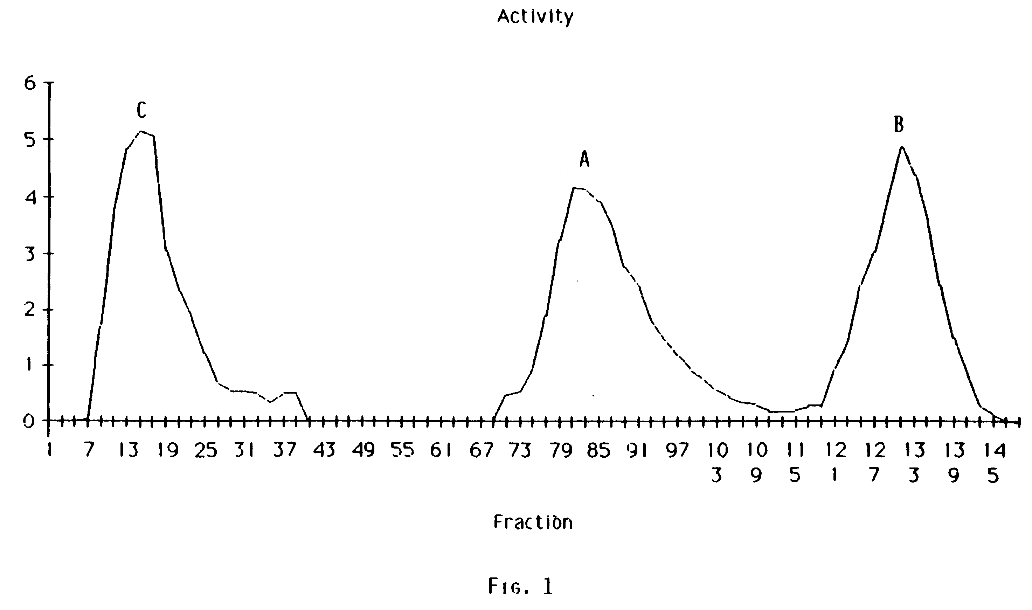

Another aspect of the present invention provides a protease preparation comprising at least 10% by activity in CPU of a constituent protease A which is immunochemically identical to the protease identified by the position of peak A in fig. 1 of the accompanying drawings and has the following additional characteristics: a molecular weight (SPS-PAGE) of about 41.5 kD, an isoelectric point (IEF) of about 9.3, a specific activity of at least 150 Anson Units and 250 Hemoglobin Protease Units (AU and HPU, respectively) per gm of protein, and a residual activity measured in CPU after 30 minutes at pH 9.5 in a solution of sodium tripolyphosphate (STPP, 1.75 g/l), sodium perborate (1.0 g/l), and tetraacetylethylenediamine (TAED, 0.1 g/l) of at least 80% and 60% at temperatures of 40°C and 50°C, respectively, of the residual activity at 25°C in the absence of sodium perborate and TAED and shows immunochemical non-identity of known alkaline Bacillus proteases, namely "ALCALASE" (derived from Bacillus licheniformis, with subtilisin Carlsberg as the active component), "SAVINASE" and "ESPERASE" (prepared from alkalophilic Bacillus sp. according to U.S. Patent No. 3723250) and "API-21" (U.S. Patent No. 4480037).

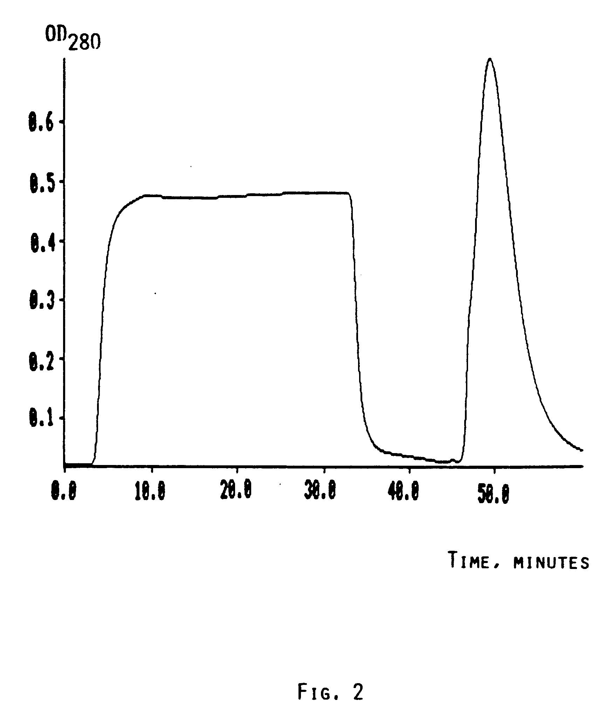

In a preferred embodiment the protease preparation is essentially devoid of proteases B and C, identified by peak B of fig. 1 and by peak C of fig. 1 and 2, respectively.

In yet another preferred embodiment of the invention the balance of the protease activity is provided jointly by said proteases B and C or singly by either of them. Immunochemically, proteases B and C are identical to each other and different from protease A. Protease B and C have molecular weights of about 28.5 and 31 kD and isoelectric points of about 8.8 and 5.2, respectively.

The present invention further provides a protease preparation comprising essentially said protease B.

In addition, the present invention is directed to a protease preparation comprising essentially said protease C.

According to a further aspect of this invention there is provided a method for producing a protease preparation, which process is characterized by cultivating a strain of the novel Bacillus species defined by the strains NCIB 12288 and NCIB 12289 under aerobic conditions in a nutrient medium containing assimilable sources of carbon, nitrogen, and phosphorus, preferably followed by recovery of the protease preparation from the fermentation broth.

In a preferred embodiment of producing the protease preparation the fermentation is conducted with a strain of the Bacillus species defined above, which strain is mutated so as to essentially block its capability of synthesizing one or two of the constituent proteases whilst maintaining its capability of producing the remaining constituent protease or proteases.

In another preferred mode of producing the protease preparation the fermentation is conducted with a Bacillus strain selected from the group consisting of NCIB 12288, NCIB 12289, NCIB 12512 and NCIB 12513.

According to yet another aspect there is provided a cleaning agent containing an effective amount, preferably at least 0.1 AU, more preferred from 0.5 to 10 AU, of the protease preparation of this invention per 100 g of cleaning agent. The cleaning agent may be a powder or a liquid wherein the protease preparation is protected, for example by incorporation into granules or as a suspension in a hydrophobic medium.

## BRIEF DESCRIPTION OF THE DRAWINGS

Fig. 1 shows the elution chromatogram of the protease preparation derived from NCIB 12289 on a CM Sepharose® CL 6B column, subsequent to gel filtration on Sephadex® G 25.

Fig. 2 shows affinity chromatography of protease C on a bacitracin column.

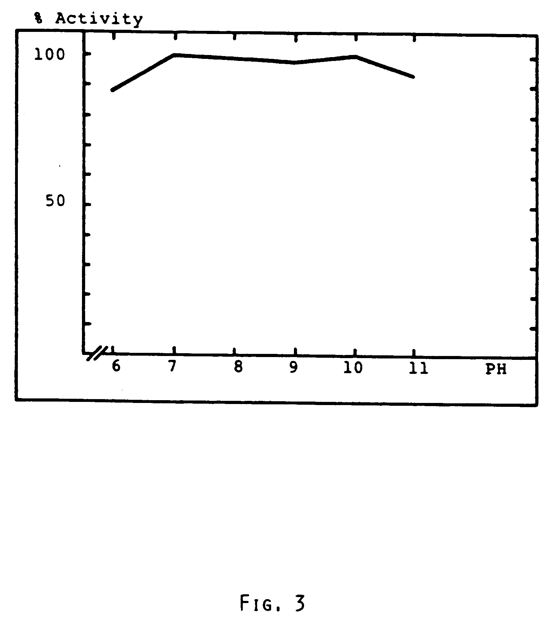

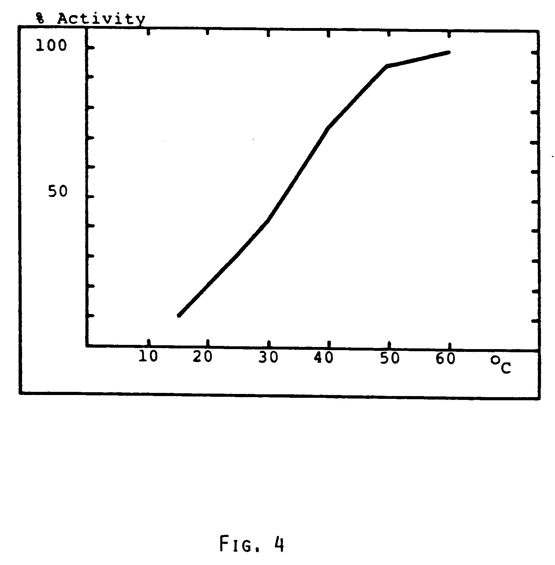

Figs. 3 and 4 are exemplary of the pH-activity and temperature-activity curves, respectively, of the protease preparation of this invention, all protease components exhibiting similar temperature- and pH-activity characteristics.

Figs. 5 and 6 illustrate the results of comparative washing experiments conducted with the protease preparation of this invention and ALCALASE using two different detergents. Experimental conditions are given in Example 5 hereinafter.

Fig. 7 illustrates the results of washing experiments conducted with each of the constituent proteases.

## DETAILED DESCRIPTION OF THE INVENTION

### The microorganism, taxonomy, morphology, biochemical reactions

The microorganism:

The microorganisms productive of the alkaline protease of the present invention were isolated essentially by the method for the selection of alkalophilic Bacilli described in British Patent No. 1,243,784. They will not grow on ordinary media for Bacillus strains unless these are supplemented by either 0.1 M carbonate buffer, pH 9.0 - 9.7 or a 7 - 10% (w/v) solution of NaCl, the 0.1 M carbonate buffer, pH 9.0 being the preferred supplement. Two cultures have been deposited with The National Collection of Industrial Bacteria (NCIB), Torry Research Station, Aberdeen, United Kingdom for the purposes of patent procedure on the date indicated below. NCIB, being an international depository authorized under the Budapest Treaty of 1977, affords permanence of the deposit and accessability thereto by the public in accordance with Rules 9 and 11, respectively, of the above treaty.

| Date of Deposit | Depositor's Reference | NCIB Designation |
|---|---|---|
| 8 July 1986 | C608 | 12288 |
| 8 July 1986 | C609 | 12289 |
| 5 August 1987 | C610 | 12512 |
| 5 August 1987 | C611 | 12513 |

Taxonomy:

Medium TY was used for taxonomic investigations which were conducted according to the methods of Ruth Gordon et al. (The Genus Bacillus, Agriculture Handbook no. 427, U.S.D.A. 1973)

Composition of medium TY:

| | |
|---|---|
| Trypticase | 20 g |
| Yeast extract | 5 g |
| $FeCl_3.6H_2O$ | 7 mg |
| $MnCl_2.4H_2O$ | 1 mg |
| $MgSO_4.7H_2O$ | 15 g |
| distilled $H_2O$ to | 1000 ml |

pH adjusted to 7.3 with KOH before autoclaving for 15 min at 121°C.

Liquid media for the determination of the production of acid from carbohydrates were supplemented with 10% NaCl before autoclaving.

Solid media were supplemented with 2% agar before autoclaving and sterile carbonate buffer pH 9.0 was aseptically added to a final concentration of 0.1 M just before pouring the slants.

The microorganisms of this invention are aerobic, spore forming bacteria belonging to the genus Bacillus.

Morphology: Vegetative rods with a diameter of 0.6 - 0.8 micron, length 1.2 - 3 micron.

Spores: Central to subterminal, oval, not swelling the sporangia.

| Biochemical reactions: | NCIB 12288 | NCIB 12289 |
| --- | --- | --- |
| Production of catalase | + | + |
| Production of acetoin (VP-test) | - | - |
| Production of indole | - | - |
| Anaerobic growth | - | - |
| Growth at 50°C | - | - |
| Growth in 7% NaCl | + | + |
| Growth at pH 5.7 | - | - |
| Hydrolysis of starch | + | + |
| Hydrolysis of hippurate | - | - |
| Hydrolysis of casein | + | + |
| Hydrolysis of tyrosine | + | + |
| Deamination of phenylalanine | - | - |
| Reduction of nitrate to nitrite | - | - |
| Acid from glucose | + | + |
| Acid from sucrose | + | + |
| Acid from xylose | + | - |
| Acid from mannitol | (+) | (+) |

None of the strains NCIB 12288 and NCIB 12289 produced acid from arabinose, mannose, melibiose, raffinose, or sorbitol.

Optimal growth conditions are 30 - 37°C, pH 9.0.

The above taxonomic results show that the two strains do not belong to any previously described species, but should be considered to define a novel Bacillus species. Taxonomic data for strains NCIB 12512 and NCIB 12513 are very similar to the above, indicating that all four strains belong to the same species.

## Assay for proteolytic activity

OPA-Casein method

Proteolytic activity is determined with casein as the substrate. One Casein Protease Unit (CPU) is defined as the amount of enzyme liberating 1 millimole of primary amino groups (determined by comparison with a serine standard) per minute under standard conditions, i.e. incubation for 30 minutes at 25°C and pH 9.5.

A 2% (w/v) solution of casein (Hammarsten, supplied by Merck A.G., West Germany) is prepared with the Universal Buffer described by Britton and Robinson (Journ.Chem.Soc. 1931, p. 1451), adjusted to pH 9.5.

Two ml of substrate solution is preincubated in a water bath for 10 minutes at 25°C. 1 ml of enzyme solution containing b g/ml of enzyme preparation, corresponding to about 0.2 - 0.3 CPU/ml of Britton-Robinson buffer (pH 9.5), is added. After 30 minutes of incubation at 25°C the reaction is terminated by the addition of a stopping agent (5 ml of a solution containing trichloroacetic acid (17.9 g), sodium acetate (29.9 g), and acetic acid (19.8 g), filled up to 500 ml with deionized water). A blank is prepared in the same manner as the test solution, except that the stopping agent is added prior to the enzyme solution.

The reaction mixtures are kept for 20 minutes in the water bath, whereupon they are filtered through Whatman® 42 paper filters.

Primary amino groups are determined by their colour development with o-phthaldialdehyde (OPA).

Disodium tetraborate decahydrate (7.62 g) and sodium dodecylsulfate (2.0 g) is dissolved in 150 ml of water. OPA (160 mg) dissolved in 4 ml of methanol is then added together with 400 $\mu$l of beta-mercaptoethanol, whereafter the solution is made up to 200 ml with water.

To the OPA reagent (3 ml) is added 400 $\mu$l of the above-mentioned filtrates with mixing. The optical density (OD) at 340 nm is measured after about 5 minutes.

The OPA test is also performed with a serine standard containing 10 mg of serine in 100 ml of Britton-Robinson buffer (pH 9.5). The buffer is used as a blank.

The protease activity is calculated from the optical density measurements by means of the following formula:

$$CPU/ml \ of \ enzyme \ solution = \frac{(OD_t - OD_b) \times C_{ser} \times Q}{(OD_{ser} - OD_B) \times MW_{ser} \times t_i}$$

CPU/g of enzyme preparation = CPU/ml: b

wherein $OD_t$, $OD_b$, $OD_{ser}$ and $OD_B$ is the optical density of the test solution, blank, serine standard, and buffer, respectively, $C_{ser}$ the concentration of serine in mg/ml in the standard, $MW_{ser}$ the molecular weight of serine. Q is the dilution factor (in this instance equal to 8) for the enzyme solution, and $t_i$ is the incubation time in minutes.

OPA-hemoglobin method

Proteolytic activity is determined with ureadenatured hemoglobin as the substrate. One Hemoglobin Protease Unit (HPU) is defined as the amount of enzyme liberating 1 millimole of primary amino groups (determined by comparison with a serine standard) per minute under standard conditions, i.e. incubation for 30 minutes at 25°C and pH 9.5.

A 2% (w/v) solution of urea-denatured hemoglobin is perpared with 0.2 M borate buffer, adjusted to pH 9.5.

Two ml of substrate solution is preincubated in a water bath for 10 minutes at 25°C. 1 ml of enzyme solution containing b g/ml of enzyme preparation, corresponding to about 0.2 - 0.3 HPU/ml of borate buffer, is added. Incubation, termination of the reaction and determination of primary amino grups by means of the OPA reagent are the same as those described above for the OPA-casein method.

The OPA test is also performed with a serine standard containing 10 mg of serine in 100 ml of the borate buffer, the buffer itself serving as a blank.

The protease activity in HPU is calculated from a formula identical to that used in the OPA-casein method.

Modified Anson-hemoglobin method

In the Anson-hemoglobin method for the determination of proteolytic acitivty, denatured hemoglobin is digested under standard conditions. The undigested hemoglobin is precipitated with trichloroacetic acid (TCA) and the amount of TCA soluble product is determined with Folin-Ciocalteu phenol reagent.

One Anson Unit (AU) is the amount of enzyme which under standard reaction conditions digests hemoglobin at such an initial rate that there is liberated per minute an amount of TCA soluble product which gives the same colour with phenol reagent as one milliequivalent of tyrosine.

The standard reaction conditions are the following: Temperature 25°C; reaction time 10 min.; pH 7.5.

For further reference, see M.L. Anson, Journal of General Physiology vol. 22 (1939), pp. 79-89; O. Folin and V. Ciocalteu, The Journal of Biological Chemistry vol. 73 (1972), pp. 627-636.

Different values for proteolytic activity will invariably be obtained depending on the choice of analytical method. While the OPA-casein assay is convenient for monitoring proteolytic activity for laboratory and production purposes, it may be said that proteolytic activity towards a porphyrin containing substrate, such as hemoglobin, is more relevant for assessing the utility of a detergent protease because of the generally experienced difficulties with removal of stains from blood and chlorophyll. A high specific activity of a detergent protease towards porphyrin containing substrates is desirable.

**The protease preparation**

The Bacillus strains productive of the protease of this invention are cultivated under aerobic conditions in a nutrient medium containing assimilable carbon and nitrogen together with other essential nutrients, the medium being composed in accordance with the principles of the known art.

Suitable carbon sources are carbohydrates, such as sucrose, glucose and starch, or carbohydrate containing materials such as cereal grains, malt, rice and sorghum. Polysaccharides are usually at least partially converted into fermentable sugars by suitable hydrolyzing enzymes prior to the fermentation process. The carbohydrate concentration incorporated in the medium may vary widely, e.g. up to 25% and down to 1 - 5%, but usually 5 - 15% will be suitable, the percentages being calculated as equivalents of glucose.

The nitrogen source in the nutrient medium may be of inorganic and/or organic nature. Suitable inorganic nitrogen sources are nitrates and ammonium salts. Among the organic nitrogen sources quite a number are regularly used in fermentation processes involving the cultivation of bacteria. Illustrative examples are soybean meal, cotton seed meal, peanut meal, casein, corn steep liquor, yeast extract, urea, and albumin. In addition, the nutrient medium should also contain the usual trace substances.

The optimum temperature for propagation of the Bacillus strains and for their production of protease is routinely ascertained by the skilled artisan. The cultivation may be conducted in the temperature range of from 25°C to 35°C, preferably at alkaline pH values. The latter may be obtained by addition of suitable buffers, such as sodium carbonate or mixtures of sodium carbonate and sodium bicarbonate after sterilization of the growth medium. For cultivation in tank fermentors it is necessary to use artificial aeration. The rate of aeration is similar to that used in conventional tank fermentation.

After fermentation, liquid enzyme concentrates may be produced by removal of coarse material from the broth and, if desired, concentration by evaporation at low temperature or by reverse osmosis. Finally, perservatives may be added to the concentrate.

Solid enzyme preparations may be prepared from the purified and/or concentrated broth by precipitation with salts, such as $Na_2SO_4$ or with water miscible solvents, such as ethanol or acetone; removal of the water in the broth by suitable drying methods such as spray-drying may also be employed.

The proteolytic activity of the protease preparation so obtained is usually in the range of from 0.1 to 10 CPU/g.

The pH-activity and temperature-activity curves of protease according to this invention are shown in fig. 1 and 2. The sample used was that prepared in example 1 below, and activity was determined by the CPU method described above, except that pH or temperature was changed. The curves show that optimum pH with casein as substrate is about 6 to 11, and that optimum temperature for 30 minutes reaction is about 50-60°C.

## Purification of Constituent Proteases

The first, second and third protease (hereinafter referred to as protease A, Protease B and protease C, respectively) may be separated and purified by separation techniques generally known by the skilled artisan.

An initial purification of the crude protease may be effected by dissolution, e.g. in a suitable buffer, followed by protein precipitation, e.g. with a salt, such as $(NH_4)_2SO_4$, or a water-miscible organic solvent, for example acetone.

Further purification of the recovered precipitate is usually attained by chromatographic methods, for example including gel filtration in a buffer system as the initial step. Protease containing fractions of the eluate may be located by monitoring U.V. absorbancy and/or by determining the protease activity.

Following gel filtration further purification is usually achieved by ion exchange chromatography, for example on a SEPHADEX® anion or cation exchanger, whereby at least partial resolution of the constituent proteases may be effected, by using suitable buffer-salt-gradient systems. Rechromatography of peak material therefrom by means of the same or a different ion exchanger usually affords the protease species in a sufficiently pure state. If required, further purification of the individual proteases may be achieved by subjecting them to affinity chromatography, for example on a bacitracin column.

Purification of the constituent proteases is conveniently monitored by gel electrophoresis, for example in a polyacrylamide gel (PAGE) with or without sodium dodecylbenzenesulphonate (SDS). Autodigestion of the proteases may be prevented by active site blocking prior to electrophoresis, for example with phenylmethylsulphonyl fluoride (PMSF).

SDS-PAGE and isoelectric focusing (IEF) in the presence of marker proteins are convenient means for molecular weight (MW) and isoelectric point (pI) determinations, respectively. According to these methods, proteases A, B and C have molecular weights of about 41.5, 28.5 and 31.0 kD, and isoelectric points of about 9.3, 8.8 and 5.2, respectively. Protease A is further characterized by specific activities per gram of protein of about 180 AU/g and 270 HPU/g.

## Immunochemical Characterization

Monospecific antisera against each of the purified proteases may be raised for example by immunizing rabbits according to the regimen described by N. Axelsen et al. in: A manual of Quantitative Immunoelectrophoresis, Blackwell Scientific Publications, 1973, chapter 23. Purified immunoglobulins may be obtained from the antisera, for example by salt precipitation $((NH_4)_2SO_4)$, followed by dialysis and ion exchange chromatography, e.g. on DEAE-Sephadex.

Immunochemical characterization of proteins is usually conducted either by Ouchterlony double-diffusion analysis (O. Ouchterlony in: Handbook of Experimental Immunology (D.M. Weir, Ed.), Blackwell Scientific Publications, 1967, pp. 655-706) or by crossed immunoelectrophoresis (N. Axelsen et al., supra, chapters 3 and 4).

Proteases B and C of the invention show immunochemical identity to each other, and both show non-identity to protease A. Proteases A, B and C of the invention all show immunochemical non-identity to the following known alkaline Bacillus proteases:

- Alcalase (product of Novo Industri A/S, Denmark), derived from Bacillus licheniformis, with Subtilisin Carlsberg as the active component.
- Savinase and Esperase (products of Novo Industri A/S), prepared from alkalophilic Bacillus sp. according to US 3,723,250.
- API-21 (U.S. patent 4,480,037).
- The protease of Danish patent application No. DK 87/0544.

## Cleaning agent

Cleaning agent according to the invention is typically a laundry detergent in liquid or powder form. In the case of a liquid detergent, the stability of the protease during storage may be unsatisfactory, and it is therefore preferred to include enzyme stabilizers in the formulation. Thus, the liquid formulation may include polyols such as 1,2-propanediol (propylene glycol), typically 1 - 20%. Optionally, the formulation may include boric acid (or alternatively, boric oxide, borax or a borak), typically up to 10%. Also optionally, formate or a salt of other lower fatty acid may be included, e.g. in an amount up to 10%. The calcium content in the liquid detergent may be from 0.001 to 0.1% (all %'es by weight).

## Example 1

### Protease preparation derived from strain NCIB 12289

Cells of Bacillus strain NCIB 12289 were harvested after 2 days' incubation at 30°C from slants of TY-agar supplemented aseptically with 0.1 M carbonate buffer pH 9.0 and 1% skim milk. The cells were transferred to 500 ml baffled shake flasks containing 100 ml BP-X medium + 0.1 M carbonate buffer pH 9.0.

Medium BP-X:

| | |
|---|---|
| potato starch | 100 g |
| barley flour | 50 g |
| soy bean flour | 20 g |
| Na-caseinate | 10 g |
| $Na_2HPO_4.12H_2O$ | 9 g |
| TERMAMYL® 60L (NOVO) | 0.1 g |
| PLURONIC™ | 0.1 g |
| tap water to 1000 ml | |

The mixture was slowly heated from 60°C to 85°C during 30 minutes with agitation in order to liquefy the starch. The mixture was then heated quickly and kept above 95°C for 10 minutes before it was cooled, distributed into the shake flasks and autoclaved for 40 minutes at 121°C.

The inoculated shake flasks were incubated for 4 days at 25°C on a rotary shaker at 240 rpm. The fermentation broth (2.5 l, 54 CPU/l, as assayed by the OPA-casein method) was centrifuged for 30 minutes at 4000 rpm. The centrifuged liquid (2.0 l) was concentrated to 1.0 l by ultrafiltration on a Mini-lab module from DDS, RO-Division, Denmark. After the concentration it was washed with 5.0 l of 0.01 molar sodium borate, pH 9.0, and finally concentrated further to 400 ml. It was then lyophilized, giving 25.5 g of crude preparation with an activity of 3.0 CPU/g. Yield: 57%.

**Example 2**

Separation of constituent proteases A, B and C

Fermentation with NCIB 12289 was performed essentially as in Example 1. The culture broth (75 AU/kg) was purified through the following steps:

- flocculation
- centrifugation
- evaporation
- blank filtration
- salt precipitation with $Na_2SO_4$
- redissolution
- blank filtration
- sterile filtration
- concentration by ultrafiltration and washing
- lyophilization

The lyophilized crude preparation (activity.15.3 CPU/g or 24.5 AU/g) was purified and separated into constituent proteases by the following sequence of steps.

1. Redissolution

2.5 g of protease was dissolved in 50 ml of the following buffer:
dimethylglutaric acid 0.01 M
boric acid 0.1 M
calcium chloride 0.002 M
sodium hydroxide to pH 7.0

2. Centrifugation

The redissolved protease was centrifuged at 18000 rpm at 25 - 30°C for 20 minutes.

3. Filtration

The supernatant was filtered on Whatman glassfilter type GF/F, 42 ml of clarified protease solution was collected.

4. Gelfiltration of Sephadex G 25 medium

40 ml of clarified protease solution was applied to a G 25 column (5 cmø x 22.6 cm) equilibrated with the buffer used in step 1. 10 ml fractions were collected, with a flow rate of 3.3 ml/min., and tested for activity. Fractions 19 - 25, comprising 54% of the applicated activity, were pooled, diluted to 100 ml and filtered on Millipore 0.45 $\mu$ filter type HA.

5. Ion exchange chromatography on CM Sepharose CL 6B

The filtered eluate was applied on a CM Sepharose CL 6B column (5 cmø x 7.8 cm) equilibrated with the buffer used in step 1. Protease C and colored substances were washed out of the column with the same buffer. The other components were eluted with a 0 - 0.05 M NaCl gradient in 2 l of the same buffer. 10 ml fractions were collected, with a flow rate of 3.3 ml/min., and monitored by UV-absorbance. The gradient was started after fraction 37. The UV-absorbing fractions 5 - 40 and 71 - 150 were tested for protease activity. The protease activity was found in 3 distinctive peaks, as seen in fig. 1.

Fractions 9 - 25, comprising 23.1% of the applicated activity, were pooled (Protease C).

Fractions 76 - 98, comprising 25.3% of the applicated activity, were pooled and designated Protease A.

Fractions 122 - 140, comprising 23.7% of the applicated activity, were pooled and designated Protease B.

10

6. Affinity chromatography on a bacitracin column

The column (1.6 cmØ x 12 cm) was equilibrated with the buffer of step 1. The pool of fractions 9 - 25 were applied on the column. The protease was eluted with the same buffer with added NaCl (1 M) and isopropanol (25%). The flow rate was 5 ml/min. and the fraction size 10 ml. The elution diagram is shown in fig. 2 (OD at 280 nm vs. time). Fractions 24 - 27 comprising 73% of the applied protease activity, were pooled and designated Protease C.

**Example 3**

Protease preparation derived from strain NCIB 12288

The same procedure as in Example 1 above was followed for the cultivation of strain NCIB 12288. The fermentation broth (0.3 l, 35 CPU/l) gave 11.7 g of crude preparation with an activity of 0.39 CPU/g. Yield: 43%.

**Example 4**

Protease preparation derived from strain NCIB 12512

The same procedure as in Example 1 above was followed for the cultivation of strain NCIB 12512. The fermentation broth (1.8 l, 30 CPU/l) gave 19.1 g of crude preparation with an activity of 1.0 CPU/g. Yield: 35%.

**Example 5**

Protease preparation derived from strain NCIB 12513

The same procedure as in Example 1 above was followed for the cultivation of strain NCIB 12513. The resulting preparation was separated as in Example 2 above and was found to be devoid of protease B.

**Example 6**

Stability of Bacillus proteases in solutions of STPP, sodium perborate and TAED

Solutions, each with an activity of 0.2 CPU per litre of the protease and containing 1.75 g/l of sodium tripoly phosphate (STPP), 1.0 g/l of sodium perborate and 0.1 g/l of tetraacetyl- ethylenediamine (TAED) were incubated for 30 minutes at 40°C and 50°C. The residual activity was then measured according to the OPA-casein method, the activity after incubation at 25°C in the absence of sodium perborate and TAED being calculated as 100%. The results are presented in the following table:

| | Percent residual activity after 30 minutes at | |
|---|---|---|
| Protease | 40°C | 50°C |
| ALCALASE | 55 - 70 | 5 - 17 |
| ESPERASE | 55 - 65 | 20 - 45 |
| SAVINASE | 40 - 50 | 0 - 10 |
| Protease of Example 1 | 90 - 100 | 60 - 75 |
| Protease of Example 3 | 95 - 100 | 70 |
| Protease A of Example 2 | 91 | 65 - 70 |

11

## Example 7

Washing Trials with Crude Protease Preparation

Washing tests were performed in a Terg-O-tometer (as described in Jay C. Harris: "Detergency Evaluation and Testing", Interscience Publishers Ltd. (1954), p. 60-61)at an agitation rate of 100 rpm for 10 minutes at 60°C with EMPA 116 test fabric swatches, 5 cm x 10 cm (cotton soiled with blood, milk, and carbon black), supplied by Eidgenössische Materialprüfungs- und Versuchsanstalt, Sankt Gallen, Switzerland. The following detergent compositions A and B were used in water of 9° German Hardness:

|  | Detergent A (g/l) | Detergent B (g/l) |
|---|---|---|
| LAS | 1.0 | 1.0 |
| Soap | 0.1 | 0.1 |
| STPP | 1.25 | 1.25 |
| Sodium silicate | 0.25 | 0.25 |
| Sodium sulphate | 2.4 | 2.4 |
| Sodium perborate | none | 1.0 |
| TAED | none | 0.1 |
| pH | 9.5 | 9.5 |

wherein
LAS is linear alkylsulfonate (NANSA 80S, produced by Albright & Wilson)
STPP is sodium tripolyphosphate
TAED is tetraacetylethylenediamine.

The cloth to liquid ratio was 9 swatches per 900 ml of detergent solution. The detergent enzymes used were the enzyme preparation of Example 1 and ALCALASE protease, both at dosage levels of 0.05, 0.1 and 0.5 CPU/l.

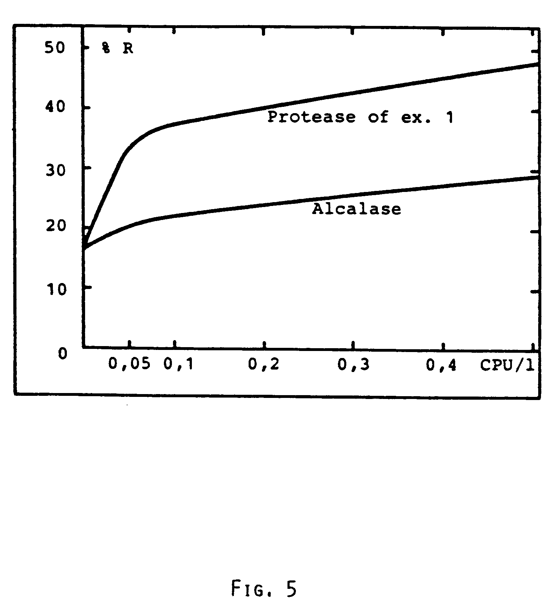

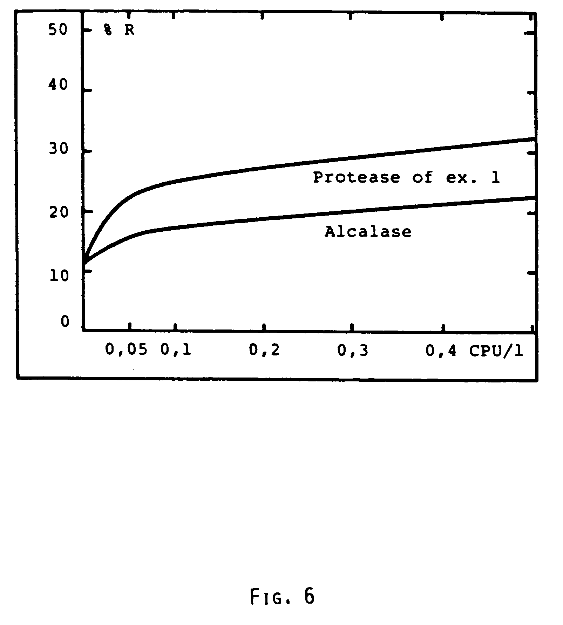

The results of duplicate washing tests with detergents A and B are shown in Fig. 5 and 6, respectively, wherein the washing efficiency is expressed as medium values of % Remission as determined by remission photometry by using a ZEISS ELREPHO photometer at 460 nm. The figure shows that the washing effect of the protease of this invention at dosage levels of about 0.05 CPU/l and above exceeds that of the ALCALASE protease both in the absence (Detergent A) and presence (Detergent B) of perborate + TAED.

## Example 8

Washing trials with constituent proteases A, B and C

Washing tests were preformed in a Terg-O-tometer at 100 rpm for 10 min at 50°C. The following detergent composition was used in water of 9° German hardness:

| LAS | 0.4 g/l |
|---|---|
| AE | 0.15 g/l |
| Soap | 0.15 g/l |
| STPP | 1.75 g/l |
| Sodium silicate | 0.4 g/l |
| CMC | 0.05 g/l |
| EDTA | 0.01 g/l |
| Sodium sulphate | 2.1 g/l |
| Sodium perborate | 1.0 g/l |
| TAED | 0.1 g/l |
| pH | 9.5 |

wherein
LAS, STPP and TAED are as in Example 7.
AE is alcohol ethoxylate (Berol 065, produced by Berol Kemi AB, Sweden).

12

Clean swatches consisted of white cotton textile, which was desized with amylase (BAN Novo), dried and cut in pieces (10 x 20 cm).

Clean swatches were immersed in spinach juice, squeezed between two rollers and dried in a tumbler at 60°C. This was repeated twice to obtain soiled swatches.

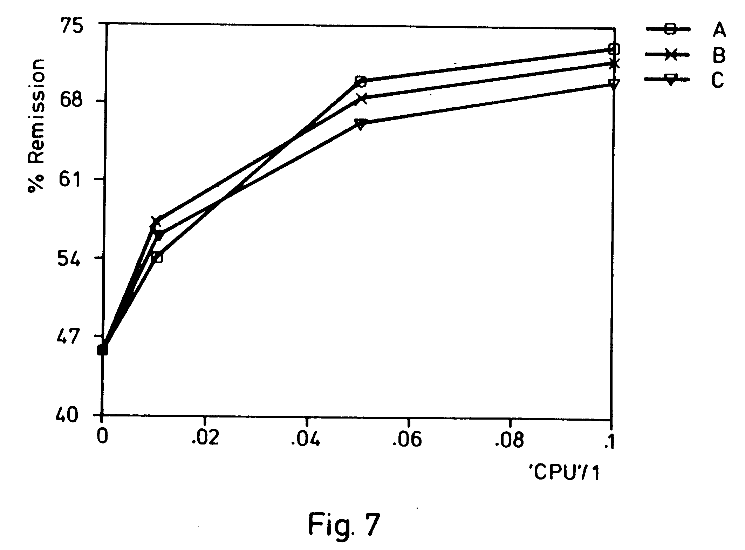

The cloth to liquid ratio was 8 swatches (4 clean and 4 soiled) in 800 ml of detergent solution. Protease A, B and C from Example 2 were tested at dosages of 0.1, 0.5 and 1.0 CPU/l. The results are shown in fig. 7 as %. Remission at 460 nm versus protease dosage.

The figure shows that all three constituent proteases have a good washing effect in detergent with perborate at 50°C.

## Example 9

Storage stability in liquid detergents

Liquid detergents were formulated as follows (amounts in g):

|  | A1/2 | B1/2 | C1/2 | D1/2 |
|---|---|---|---|---|
| **Fraction I:** | | | | |
| Water | 30.0/42.0 | 30.0/42.0 | 5.0/17.0 | 28.0/40.0 |
| TEA | 10.0 | 10.0 | 10.0 | 10.0 |
| $CaCl_2, 2H_2O$ | 0.10 | 0.10 | 0.10 | 0.10 |
| DTPA | 0.10 | 0.10 | 0.10 | 0.10 |
| DG | – | – | – | – |
| Tri-sodium Citrate | 5.0 | 5.0 | 5.0 | 5.0 |
| $H_3BO_3$ | 0/5.0 | 0/5.0 | 0/5.0 | 0/5.0 |
| **Fraction II:** | | | | |
| MPG | 0/12.5 | 0/12.5 | 0/12.5 | 0/12.5 |
| Ethanol, 96% | 7.0 | 7.0 | 7.0 | 7.0 |
| Oleic acid | 5.0 | 5.0 | 5.0 | 5.0 |
| AE | 15.0 | 15.0 | 15.0 | 15.0 |
| AES | 25.0 | 25.0 | – | 25.0 |
| LAS | – | – | 50.0 | – |
| 10 N NaOH to pH: | 9.0 | 10.0 | 9.0 | 9.0 |
| Water added to (g): | 100/129.7 | 100/129.7 | 100/129.7 | 100/129.7 |

TEA stands for triethanolamine, DTPA for penta-sodium salt of diethylene-triamine-pentaacetate, DG for sodium diglycolate, MPG for mono-propylene glycol, AES for alcohol-ethoxy sulphate (Dobanol 25-3S/60, product of Shell), LAS for linear alkyl-benzene sulphonate (Nansa 1169P), and AE stands for alcohol ehtoxylate (Berol 160, product of Berol Kemi AB, Sweden). In each case, the ingredients in fraction I and II were mixed, and then fraction II was added slowly to fraction I while stirring.

The water content (including water contained in other ingredients) was about 41% w/w in all detergents.

The following protease preparations were tested:

- The lyophilized crude preparation of Example 2, dissolved in a mixture of water and MPG (1:1 by weight) and diluted to an activity of 2.5 AU/g.
- Alcalase 2.5L (product of Novo Industri A/S, Denmark), a commercial liquid preparation of alkaline protease from Bacillus licheniformis.
- Esperase 8.0L (product of Novo Industri A/S, Denmark), a commercial liquid preparation of alkaline Bacillus protease prepared according to US 3,723,250.

The calcium content of each protease preparation was adjusted to 1.0% w/w (as $Ca^{++}$) by addition Of $CaCl_2$, $2H_2O$. In each experiment, 1% w/w of a protease preparation (after $Ca^{++}$ adjustment) was added to one of the above detergents, the mixture was incubated at 50°C, and samples were taken after 0, 23, 47 and 134 hours incubation. The protease activity of each sample was determined, and the results are shown below, expressed as residual activity in % of initial activity (i.e. activity after 0 days). pH was measured after 0 and 134 hours.

pH:

| | 0 h | 134 h | 0 h | 134 h |
|---|---|---|---|---|
| Detergent | A1 | | A2 | |
| Ex. 2 | 9.0 | 9.0 | 9.0 | 9.0 |
| ESPERASE | 9.0 | 9.1 | 9.0 | 9.0 |
| ALCALASE | 9.0 | 9.1 | 8.9 | 9.0 |
| | | | | |
| Detergent | B1 | | B2 | |
| Ex. 2 | 10.0 | 9.9 | 9.9 | 9.9 |
| ESPERASE | 10.1 | 9.8 | 9.9 | 9.8 |
| ALCALASE | 10.1 | 9.8 | 9.9 | 9.8 |
| | | | | |
| Detergent | C1 | | C2 | |
| Ex. 2 | 9.2 | 9.2 | 9.0 | 9.0 |
| ESPERASE | 9.2 | 9.2 | 9.0 | 9.0 |
| ALCALASE | 9.2 | 9.2 | 9.0 | 9.1 |
| | | | | |
| Detergent | D1 | | D2 | |
| Ex. 2 | 9.1 | 9.2 | 9.1 | 9.0 |
| ESPERASE | 9.2 | 9.1 | 9.1 | 9.0 |
| ALCALASE | 9.2 | 9.1 | 9.1 | 9.0 |

% residual activity:

|  | 23 h | 47 h | 134 h |  | 23 h | 47 h | 134 h |
| --- | --- | --- | --- | --- | --- | --- | --- |
| Detergent | | A1 | | | | A2 | |
| Ex. 2 | 46 | 37 | 16 | | 102 | 90 | 86 |
| ESPERASE | 3 | 2 | 1 | | 72 | 77 | 53 |
| ALCALASE | < 1 | < 1 | < 1 | | 1 | 1 | < 1 |
| Detergent | | B1 | | | | B2 | |
| Ex. 2 | 43 | 33 | 12 | | 91 | 80 | 55 |
| ESPERASE | 2 | 2 | < 1 | | 67 | 70 | 39 |
| ALCALASE | < 1 | < 1 | < 1 | | < 1 | < 1 | < 1 |
| Detergent | | C1 | | | | C2 | |
| Ex. 2 | 38 | 31 | 16 | | 89 | 80 | 66 |
| ESPERASE | 2 | 2 | 21 | | 73 | 72 | 41 |
| ALCALASE | < 1 | < 1 | < 1 | | 15 | 13 | 3 |
| Detergent | | D1 | | | | D2 | |
| Ex. 2 | 46 | 37 | 19 | | 92 | 87 | 75 |
| ESPERASE | 9 | 5 | 1 | | 90 | 88 | 66 |
| ALCALASE | < 1 | < 1 | < 1 | | 4 | 4 | 1 |

pH was nearly constant in all experiments. It is seen that protease of the invention is more stable than Esperase and Alcalase in all the detergent formulations tested. The results further show that borate and MPG are very effective as stabilizers for protease of the invention in liquid detergents.

**Claims**

1. A protease preparation comprising alkaline protease derivable from a novel alkalophilic Bacillus species defined by the strain NCIB 12288 and NCIB 12289 which alkaline protease retains a residual activity measured in Casein Protease Units (CPU) after 30 minutes at pH 9.5 in a solution of 1.75 g/l of sodium tripolyphosphate (STPP), 1.0 g/l of sodium perborate and 0.1 g/l of tetraacetylethylenediamine (TAED) of at least 80% and 60% at temperatures of 40°C and 50°C, respectively, of the residual activity at 25°C in the absence of sodium perborate and TAED, the protease having a molecular weight (SPS-PAGE) of about 41.5 kD and an isoelectric point (IEF) of about 9.3, a molecular weight (SPS-PAGE) of about 28.5 kD and an isoelectric point (IEF) of about 8.8 or a molecular weight (SPS-PAGE) of about 30.0 kD and an isoelectric point (IEF) of about 5.2, the protease showing immunochemical non-identity to the following alkaline Bacillus proteases, namely "ALCALASE", "SAVINASE", "ESPERASE" and "API-21".

2. A protease preparation comprising at least 10% by activity in CPU of a constituent protease A which is immunochemically identical to the protease identified by the position of peak A in fig. 1 of the accompanying drawings and has the following additional characteristics: a molecular weight (SPS-PAGE) of about 41.5 kD, an isoelectric point (IEF) of about 9.3, a specific activity of at least 150 Anson Units and 250 Hemoglobin Protease Units (AU and HPU, respectively) per gm of protein, and a residual activity measured in CPU after 30 minutes at pH 9.5 in a solution of sodium tripolyphosphate (STPP, 1.75 g/l), sodium perborate (1.0 g/l), and tetraacetylethylenediamine (TAED, 0.1 g/l) of at least 80% and 60% at temperatures of 40°C and 50°C, respectively, of the residual activity at 25°C in the absence of sodium perborate and TAED and shows immunochemical non-identity to the following alkaline Bacillus proteases, namely "ALCALASE", "SAVINASE", "ESPERASE" and "API-21".

3.  The protease preparation of Claim 2 which is essentially devoid of other proteases than said first protease A.

4.  The protease preparation of Claim 2, wherein the balance of the protease activity is provided jointly by proteases B and C or singly by either of these, said proteases being identified by peak B of fig. 1 and by peak C of fig. 1 and 2, by molecular weights (SPS-PAGE) of about 28.5 and 31.0 kD by isoelectric points (IEF) of about 8.8 and 5.2, respectively, said proteases B and C being immunochemically identical to each other, but non-identical to said protease A.

5.  A protease preparation wherein the protease activity is essentially provided by said protease B as defined in Claim 4.

6.  A protease preparation wherein the protease activity is essentially provided by said protease C as defined in Claim 4.

7.  A method for producing a protease preparation, which process is characterized by cultivating a strain of the novel Bacillus species defined by the strains NCIB 12288 and NCIB 12289 under aerobic conditions in a nutrient medium containing assimilable sources of carbon, nitrogen, and phosphorus, preferably followed by recovery of the protease preparation from the fermentation broth.

8.  The method of Claim 7, wherein the fermentation is conducted with a strain of the Bacillus species defined above, which strain is mutated so as to essentially block its capability of synehesizing one or two of the constituent proteases whilst maintaining its capability of producing the remaining constituent protease or proteases.

9.  The method of Claim 7, wherein the fermentation is conducted with a Bacillus strain selected from the group consisting of NCIB 12288, NCIB 12289, NCIB 12512 and NCIB 12513.

10.  A cleaning agent containing an effective amount, preferably at least 0.1 AU, more preferred from 0.5 to 10 AU, of the protease preparation of any one of the Claims 1 to 6 and/or protease preparation produced by the method of any one of the Claims 7 to 9.

**Patentansprüche**

1.  Proteasezubereitung, die von einer neuartigen alkalophilen Bacillus-Spezies, definiert durch den Stamm NCIB 12288 und NCIB12289, ableitbare Alkalinprotease umfaßt, wobei die Alkalinprotease eine Restaktivität, gemessen in Casein Protease Units (CPU) nach 30 Minuten bei pH 9,5 in einer Lösung von 1,75 g/l Natriumtripolyphosphat (STPP), 1,0 g/l Natriumperborat und 0,1 g/l Tetraacetylethylendiamin (TAED), von wenigstens 80 % bzw. 60 % bei Temperaturen von 40°C bzw. 50°C der Restaktivität bei 25°C in der Abwesenheit von Natriumperborat und TAED beibehalt, wobei die Protease ein Molekulargewicht (SPS-PAGE) von etwa 41,5 kD und einen isoelektrischen Punkt (IEF) von etwa 9,3, ein Molekulargewicht (SPS-PAGE) von etwa 28,5 kD und einen isoelektrischen Punkt (IEF) von etwa 8,8 oder ein Molekulargewicht (SPS-PAGE) von etwa 30,0 kD und einen isoelektrischen Punkt (IEF) von etwa 5,2 besitzt, wobei die Protease immunchemische Nicht-Identität zu bekannten alkalischen Bacillus-Proteasen zeigt, nämlich "ALCALASE", "SAVINASE", "ESPERASE" und "API-21".

2.  Proteasezubereitung, die wenigstens 10 % nach Aktivität in CPU einer konstituierenden Protease A umfaßt, die immunchemisch identisch zu der durch die Position von Peak A in Fig. 1 der beigefügten Zeichnungen identifizierten Protease ist und die folgenden zusätzlichen Merkmale besitzt: ein Molekulargewicht (SPS-PAGE) von etwa 41,5 kD, einen isoelektrischen Punkt (IEF) von etwa 9,3, eine spezifische Aktivität von wenigstens 150 Anson Units und 250 Hemoglobin Protease Units (AU bzw. HPU) pro g Protein und eine Restaktivität, gemessen in CPU, nach 30 Minuten bei pH 9,5 in einer Lösung von Natriumtripolyphosphat (STPP, 1,75 g/l), Natriumperborat (1,0 g/l) und Tetraacetylethylendiamin (TAED, 0,1 g/l) von wenigstens 80 % bzw. 60 % bei Temperaturen von 40°C bzw. 50°C der Restaktivität bei 25°C in der Abwesenheit von Natriumperborat und TAED und immunchemische Nicht-Identität mit bekannten alkalischen Bacillus-Proteasen zeigt, nämlich "ALCALASE", "SAVINASE", "ESPERASE" und "API-21".

3. Proteasezubereitung nach Anspruch 2, die im wesentlichen frei von anderen Proteasen als besagter erster Protease A ist.

4. Proteasezubereitung nach Anspruch 2, wobei der Rest der Proteaseaktivität durch die Proteasen B und C zusammen oder einzeln durch eine von diesen bereitgestellt wird, wobei besagte Proteasen durch Peak B von Fig. 1 bzw. durch Peak C von Fig. 1 und 2, durch Molekulargewichte (SPS-PAGE) von etwa 28,5 bzw. 31,0 kD, durch isoelektrische Punkte (IEF) von etwa 8,8 bzw. 5,2 identifiziert sind, wobei besagte Proteasen B und C immunchemisch identisch zueinander sind, aber verschieden von besagter Protease A.

5. Proteasezubereitung, die im wesentlichen besagte Protease B, wie definiert in Anspruch 4, umfaßt.

6. Proteasezubereitung, die im wesentlichen besagte Protease C, wie definiert in Anspruch 4, umfaßt.

7. Verfahren zur Herstellung einer Proteasezubereitung, welches gekennzeichnet ist durch Kultivierung eines Stammes der neuartigen Bacillus-Spezies, die durch die Stämme NCIB 12288 und NCIB 12289 definiert ist, unter aeroben Bedingungen in einem Nährstoffmedium, das assimilierbare Kohlenstoff-, Stickstoff- und Phosphorquellen enthält, vorzugsweise gefolgt von Gewinnung der Proteasezubereitung aus der Fermentationsbrühe.

8. Verfahren nach Anspruch 7, wobei die Fermentation mit einem Stamm der oben definierten Bacillus-Spezies durchgeführt wird, welcher so mutiert ist, daß seine Fähigkeit zur Synthese von ein oder zwei der konstituierenden Proteasen im wesentlichen blockiert ist, während seine Fähigkeit, die übrigbleibende(n) konstituierende(n) Protease oder Proteasen zu produzieren, beibehalten ist.

9. Verfahren nach Anspruch 7, wobei die Fermentation mit einem Bacillus-Stamm durchgeführt wird, der ausgewählt ist aus der Gruppe, die aus NCIB 12288, NCIB 12289, NCIB 12512 und NCIB 12513 besteht.

10. Reinigungsmittel, das eine wirksame Menge, vorzugsweise wenigstens 0,1 AU, bevorzugter von 0,5 bis 10 AU, der Proteasezubereitung einer der Ansprüche 1 bis 6 und/oder der Proteasezubereitung, die nach dem Verfahren einer der Ansprüche 7 bis 9 hergestellt ist, enthält.

**Revendications**

1. Une préparation de protéase comprenant une protéase alcaline pouvant être obtenue à partir d'une nouvelle espèce alcalophile de Bacillus définie par les souches NCIB 12288 et NCIB 12289, laquelle protéase alcaline conserve une activité résiduelle mesurée en Unités de Protéase Caséine (UPC), après 30 minutes à pH 9,5 dans une solution à 1,75 g/l de tripolyphosphate de sodium (STPP), 1,0 g/l de perborate de sodium et 0,1 g/l de tétraacétyléthylènediamine(TAED), d'au moins 80 % et 60 %, à des températures respectivement de 40°C et de 50°C, de l'activité résiduelle à 25°C en l'absence de perborate de sodium et de TAED, la protéase ayant un poids moléculaire (SDS-PAGE) d'environ 41,5 kD et un point isoélectrique (pI) d'environ 9,3, un poids moléculaire (SDS-PAGE) d'environ 28,5 kD et un point isoélectrique (pI) d'environ 8,8 ou un poids moléculaire (SDS-PAGE) d'environ 30,0 kD et un point isoélectrique (pI) d'environ 5,2, la protéase présentant une non-identité immunochimique relativement aux protéases alcalines connues de Bacillus, c'est-à-dire l'"ALCALASE", la "SAVINASE", l'"ESPERASE" et l'"API-21".

2. Une préparation de protéase comprenant au moins 10 % d'activité en UPC d'un constituant protéase A qui est immunochimiquement identique à la protéase identifiée par la position du pic A de la figure 1 des dessins annexés et a les caractéristiques additionnelles suivantes : un poids moléculaire (SDS-PAGE) d'environ 41,5 kD, un point isoélectrique (pI) d'environ 9,3, une activité spécifique d'au moins 150 Unités Anson et 250 unités de Protéase Hémoglobine (respectivement UA et UPH) par gramme de protéine, une activité résiduelle mesurée en UPC, après 30 minutes à pH 9,5 dans une solution de tripolyphosphate de sodium (STPP ; 1,75 g/l), de perborate de sodium (1,0 g/l) et de tétraacétyléthylènediamine (TAED ; 0,1 g/l), d'au moins 80 % et 60 %, à des températures respectivement de 40°C et de 50°C, de l'activité résiduelle à 25°C en l'absence de perborate de sodium et de TAED, et présente une non-identité immunochimique vis-à-vis des protéases alcalines connues de Bacillus, c'est-à-dire l'"ALCALASE", la "SAVINASE", l'"ESPERASE" et l'"API-21".

3. La préparation de protéase de la revendication 2 qui est essentiellement dépourvue de protéases autres que ladite première protéase A.

4. La préparation de protéase de la revendication 2, dans laquelle le reste de l'activité protéasique est fourni conjointement par des protéases B et C ou uniquement par l'une ou l'autre d'entre elles, lesdites protéases étant respectivement identifiées par le pic B de la figure 1 et par le pic C des figures 1 et 2, par des poids moléculaires (SDS-PAGE) d'environ 28,5 et 31,0 kD et par des points isoélectriques (pI) d'environ 8,8 et 5,2, lesdites protéases B et C étant immunochimiquement identiques entre elles, mais différentes de ladite protéase A.

5. Une préparation de protéase comprenant essentiellement ladite protéase B, comme défini dans la revendication 4.

6. Une préparation de protéase comprenant essentiellement ladite protéase C, comme défini dans la revendication 4.

7. Un procédé pour la production d'une préparation de protéase, lequel procédé est caractérisé par la culture d'une souche de la nouvelle espèce de Bacillus définie par les souches NCIB 12288 et NCIB 12289, dans des conditions aérobies, dans un milieu nutritif contenant des sources assimilables de carbone, d'azote et de phosphore, de préférence suivie de la récupération de la préparation de protéase à partir du bouillon de fermentation.

8. Le procédé de la revendication 7, dans lequel la fermentation est effectuée avec une souche de l'espèce Bacillus définie ci-dessus, laquelle souche a été soumise à une mutation afin de bloquer essentiellement sa capacité à synthétiser une ou deux des protéases constitutives, tout en conservant sa capacité à produire la ou les protéases constitutives restantes.

9. Le procédé de la revendication 7, dans lequel la fermentation est effectuée avec une souche de Bacillus choisie dans le groupe constitué par NCIB 12288, NCIB 12289, NCIB 12512 et NCIB 12513.

10. Un agent de nettoyage contenant une quantité efficace, de préférence au moins 0,1 UA, mieux 0,5 à 10 UA, de la préparation de protéase de l'une quelconque des revendications 1 à 6 et/ou une préparation de protéase produite selon le procédé de l'une quelconque des revendications 7 à 9.

Fig. 1

FIG. 2

FIG. 3

% Activity

Fig. 4

FIG. 5

FIG. 6

24

Fig. 7